# EUROPEAN PATENT SPECIFICATION

(11) **EP 0 313 972 B1**
(45) Date of publication and mention of the grant of the patent: **02.02.1994**
(21) Application number: 88117365.2
(22) Date of filing: 19.10.1988
(51) Int. Cl.: G01N 33/28

(54) **Analytical method and apparatus for analyzing liquid substances**
Analyseverfahren und Vorrichtung zur Analyse von flüssigen Stoffen
Méthode d'analyse et appareil pour analyser des substances liquides

(30) Priority: 30.10.1987 JP 273417/87
(43) Date of publication of application: 03.05.1989
(73) Proprietor: IDEMITSU KOSAN COMPANY LIMITED, Tokyo 100 (JP)
(72) Inventor: Ikeda, Makoto, c/o Idemitsu Kosan Co.Ltd., Ichihara-shi, Chiba-ken (JP)
(74) Representative: Türk, Gille, Hrabal, Leifert

(56) References cited:
- DE-A- 2 019 228
- FR-A- 2 532 429
- US-A- 3 654 145
- PATENT ABSTRACTS OF JAPAN, vol. 8, no. 137 (P-282)[1574], 26 June 1984#

## Description

This invention relates to a method of and an apparatus for analyzing liquid substances, and more particularly to a method of and an apparatus for analyzing liquid substances such as a liquid substance like lubricating oil, the qualities of which may change as the time proceeds, whereby the modification or service life of the substance can be estimated or foreseen simply and precisely.

Conventionally, for estimating or foreseeing the modification and service life of chemical substances the qualities of which may change as the time proceeds, like lubricating oils during use, fats and oils during storage, and so on, various chemical and physical analytical methods have been employed to measure the neutralization values and the viscosities of the substances.

Although the conventional methods are useful to know whether chemical substances have been modified, degraded or not, they have no effect estimating or foreseeing the service life of the chemical substances.

In like manner, though infrared spectrography, gas chromatography, and the like are useful to elucidate the chemical structures of chemical substances, they are inefficient in estimating and foreseeing the modification and the service life of chemical substances. Moreover, it is impossible to analyze both of the light and heavy fractions of a chemical substance using only these analytical methods.

In brief, by any of the conventional analytical methods, it is very difficult to estimate and foresee the modification and the service life of a chemical substances in which the qualities may change with the passage of time, though it is generally recognized to be of great importance.

In view of the foregoing, the inventor has intensively studied to develop an analytical method and apparatus for analyzing a substance which, by surpassing the above-mentioned disadvantages of conventional analytical methods, realize the foreseeing or quickly recognizing the modification and service life of a liquid chemical substance such as a lubricating oil, etc., in which the qualities may change with the passage of time. As a result of the extensive study, it has been found that the foreseeing or quickly recognizing as described above is facilitated by separating the liquid chemical substance at the proper temperature and pressure into light and heavy fractions, namely vapor and liquid fractions, and analyzing both fractions simultaneously.

The present invention relates to a method of analyzing substances which comprises sampling a predetermined amount of a liquid sample, bringing the sample to a vapor-liquid equilibrium at a predetermined temperature and pressure, separating the gaseous and liquid fractions from each other, and analyzing the gaseous fraction as a gas sample and the liquid fraction as a liquid sample.

The present invention relates also to an analytical apparatus, comprising a sampling portion in which a predetermined amount of a liquid sample is collected, a vapor-liquid equilibrium portion in which the sample is brought to a vapor-liquid equilibrium under conditions of temperature and pressure controlled to predetermined values, and a vapor-liquid separation portion in which the gaseous and liquid fractions produced in the vapor-liquid equilibrium portion are separated from each other for separately analyzing each.

In the drawings
Fig. 1 is a system diagram of a preferred embodiment of an analytical apparatus of the invention, and
Fig. 2 is a graph illustrating gas chromatographic analytical results obtained according to the invention.

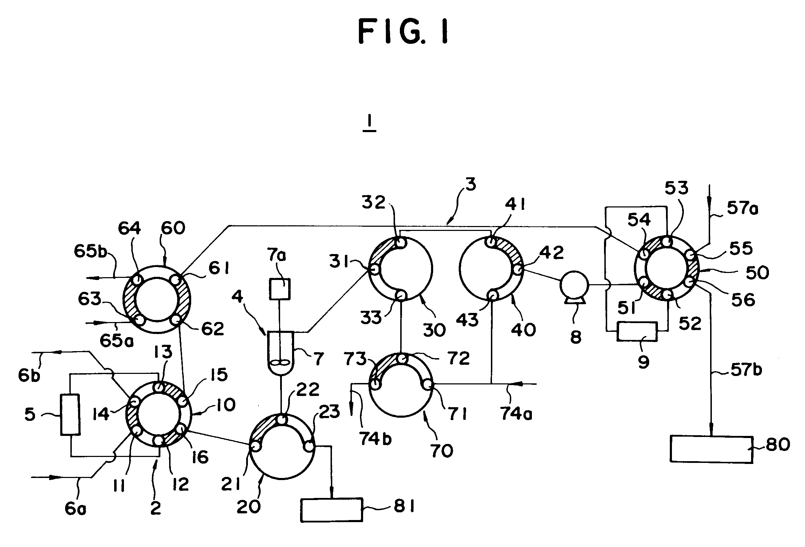

In Fig 1, an analytical apparatus 1 comprises a sampling portion 2, a vapor-liquid equilibrium portion 3, a vapor-liquid separation portion 4, a sampling column by 5, a vapor-liquid separation vessel 7, a gas sampling column by 9, six-way valves by 10 and 50, cross valves 20, 30, 40, and 70, a gas analytical apparatus 80, and a liquid analytical apparatus 81.

Hereinafter, the present invention will be described, making reference to Fig. 1. The analytical method of the invention may be carried out by using various techniques. Preferably, the present analytical method is most efficiently and precisely conducted with the present apparatus for analyzing substances.

In Fig. 1, the analytical apparatus 1 consists of a sampling portion 2 in which a predetermined amount of a sample is collected, a vapor-liquid equilibrium portion 3 in which the sample is brought to a vapor-liquid equilibrium by heating to a predetermined temperature and controlling pressure to a predetermined value, and a vapor-liquid separation portion 4 in which the gaseous and liquid fractions of the sample produced in the vapor-liquid equilibrium portion 3 are separated from each other. Each portion is placed in a constant temperature air bath, in which the temperature is controlled at predetermined levels in relationship to each action of the portions.

The sampling portion 2 consists of a six-way valve 10, a sampling column 5, a sample introduction pipe 6a, and a sample discharge pipe 6b. The vapor-liquid equilibrium portion 3 consists of a closed flow-path having the vapor-liquid separation vessel 7 of the vapor-liquid equilibrium portion 4 incorporated therein. More particularly, the closed flow-path is constituted by the above-described six-way valve 10, a cross valve 20, the vapor-liquid separation vessel 7, a cross valve 30, a cross valve 40, a circulation pump 8, a six-way valve 50, a gas sampling column 9, a four-way valve 60, and a piping for connecting the valves and so forth.

The above-described four-way valve 60 is provided with pipings 65a and 65b for introducing or discharging a solvent for diluting the sample. The analytical apparatus 1 is provided with a cross valve 70, pipings 74a, 74b for introducing and discharging a purge gas to maintain the interior of analytical apparatus 1 at a standard state, and to clean or dry the interior of the apparatus 1. The six-way valve 50 is provided with pipings 57a, 57b for introducing and discharging a carrier gas into and out of the closed flow-path.

The analytical apparatus 1 of the invention, which has such a constitution as described above, is applied to the analysis of a sample by operating the valves in the following order.

First, the valves and pipings within analytical apparatus 1 are brought to a predetermined temperature. In this state, the six-way valve 10 is switched to the sampling side so that a sample is delivered into the sampling column 5. More particularly, the sample flows through the sample introduction pipe 6a, valve opening 11, valve opening 12, sampling column 5, valve opening 13, valve opening 14, and sample discharge pipe 6b in that order. As a result, a predetermined amount of the sample flows into the sampling column 5.

With these valves operating, circulation pump 8 is operated so that the sample is circulated with purge gas adjusted to a predetermined pressure through the closed flow-path in the vapor-liquid equilibrium portion. More particularly, the purge gas is circulated from circulation pump 8, through valve opening 51, valve opening 52, gas sampling column 9, valve opening 53, valve opening 54, valve opening 61, valve opening 62, valve opening 15, valve opening 13, sampling column 5, valve opening 12, valve opening 16, valve opening 21, valve opening 22, vapor-liquid separation vessel 7, valve opening 31, valve opening 32, valve opening 41, valve opening 42, and to circulation pump 8. The sample is fed with and subjected to bubbling with purge gas in vapor-liquid separation vessel 7 to produce a gaseous fraction, which is circulated through the closed flow-path from vapor-liquid separation vessel 7. But, the liquid fraction of the sample remains in the vapor-liquid separation vessel 7.

After a predetermined period of time, circulation is stopped. Then, carrier gas is made to flow through valve opening 55, valve opening 53, gas sampling column 9, valve opening 52, and valve opening 56, so that the gaseous fraction is fed into a gas analytical apparatus 80 such as a gas chromatograph, and so on.

A dilution solvent supplied from piping 65a is introduced into the vapor-liquid separation vessel 7 via valve opening 63, valve opening 62, valve opening 15, valve opening 13, sampling column 5, valve opening 12, valve opening 16, valve opening 21, and valve opening 22, which dilutes the liquid fraction. The diluted liquid fraction is then fed to an apparatus for analyzing liquids 81 such as a liquid chromatograph, and so forth through valve opening 22 and valve opening 23.

As seen from the above description, the analytical apparatus 1 has such a constitution that the switching of each valve, the operation of the pump, the supply of a carrier gas and dilution solvent can be regulated on a definite schedule. This facilitates analysis in which a predetermined amount of a sample is allowed to come to a vapor-liquid equilibrium at predetermined temperature and pressure, and is separated into gaseous and liquid fractions, as gas and liquid analytical samples. Moreover, each operation can be automated, which enhances the reproducibility and reliability of analytical results.

Regarding the gas and liquid analytical apparatus for the invention, analytical apparatus generally used for such purposes may be properly selected, according to the type of liquid and gaseous samples to be analyzed. Typical gas analytical apparatus such as a gas chromatograph-mass spectrometer and the like are exemplified in addition to the above-described gas chromatograph. Liquid analytical apparatus such as an infrared spectroscope, ultra-violet and visible ray spectroscopes, and so forth may be properly selected, depending on the type of samples.

To prepare the next sample, in analytical apparatus 1 after sampling, a purge gas such as nitrogen gas, helium gas, or the like is introduced through piping 74a via valve opening 43. It is made to flow through each valve and pipe in the closed flow-path, and is discharged through piping 74b via valve openings 72 and 73 to dry the interior of the valves and the pipes.

As described above, according to the present invention, a sample is separated into gaseous and liquid fractions which are analyzed respectively. This enables the light and heavy fractions of a substance to be simultaneously analyzed in composition and chemical structure. Moreover, the modification and the service life of a chemical substance such as a lubricating oil in which the qualities may change with the passage of time can be estimated or foreseen. The present analytical method and apparatus for analyzing a substance are of high practical usefulness, and are expected to be used in diversified fields.

The invention will be more clearly understood with reference to the following example.

### Example

The samples used for the analysis were a lubricating oil (sample ① having a viscosity of 400 cSt. at 40°C and a total acid value of 0.1 mg KOH/g, and samples (sample ②, ③, and ④, respectively) prepared by pyrolysis of the sample ① at high temperature for 24 hours, 120 hours and 240 hours. For pyrolysis at high temperature, water equal to 5% by weight was added to the samples which were then heated at 300°C in an autoclave, and pressurized at 15 kg/cm².

The separation of each sample was conducted at 60° C and 0 kg/cm² in pressure (gauge pressure) by using helium gas as a carrier gas. The obtained gaseous fraction was analyzed with a gas chromatograph, and the liquid fraction with a liquid chromatograph.

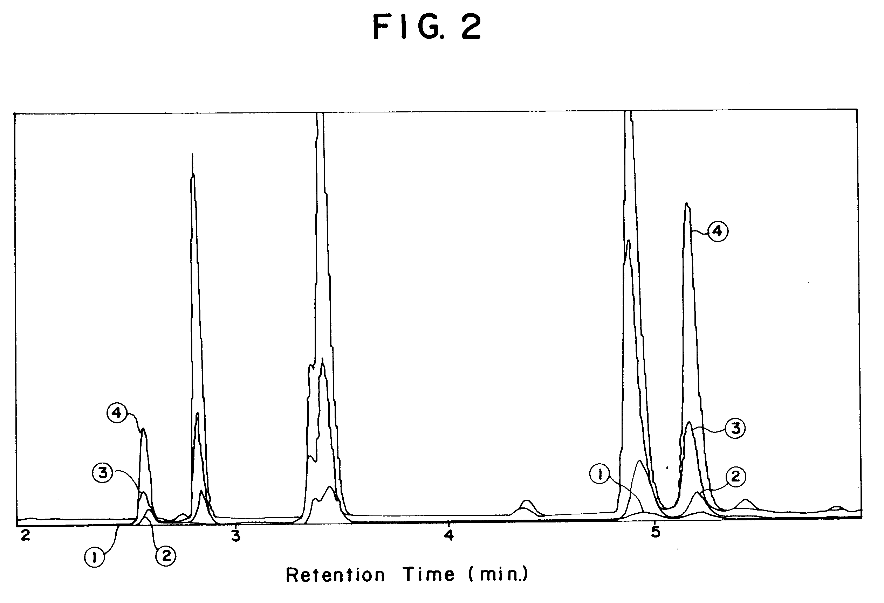

Fig. 2 illustrates the analytical results of the gaseous fraction of each sample. The peaks exhibited sequentially from left to right in each chromatogram were due to methane, ethane, propane, isobutene, isobutane and n-butane, respectively. The peaks of the sample ④ were higher than those of the sample ①. This revealed that the amount of the light fractions such as methane, ethane and the like increased as pyrolysis at a high temperature was prolonged.

The analytical results of the liquid fractions were as follows: The results described below are relative molecular weights based on 100 being the molecular weight of the liquid fraction of sample ①.

| | |
|---|---|
| Sample ① | 100 |
| Sample ② | 90 |
| Sample ③ | 87 |
| Sample ④ | 82 |

The analytical result revealed that the molecular weight of the samples decreased as high temperature pyrolysis time was longer.

Based on the analytical results, the modification rate of the lubricating oil can be estimated definitely. That is, the ratio of the peak heights of sample ④ to those of sample ② or the peak heights of sample ④ to those of sample ③ is determined, and is taken as the generation rate of the decomposition gases. Also, the rate of the viscosity change of the liquid fractions is determined by using the changes in molecular weight of the liquid fractions. The generation rate of the decomposition gases and the rate of the viscosity changed are used for the estimation of the modification rate of the lubricating oil.

By comparison with the recorded analytical-values of a sample practically used, the service life of the same kind of a sample can be estimated more definitely.

## Claims

1. Method of analyzing liquid substances like lubricant oil, comprising sampling a predetermined amount of a liquid sample, bringing the sample to a vapor-liquid equilibrium at a predetermined temperature and pressure by bubbling purge gas in a vapor-liquid separation vessel to produce a gaseous fraction with said purge gas circulating through a closed flow path, separating the resultant gaseous and liquid fractions from each other by expelling the gaseous fraction by stopping the circulation after a predetermined time and feeding the gaseous fraction by inflowing carrier gas into a gas analytical apparatus for analyzing the gaseous fraction as a gas sample and the liquid fraction as a liquid sample.

2. Method as claimed in claim 1, characterized in that the liquid fraction is diluted before analyzing.

3. Apparatus for analyzing liquid substances like lubricant oil, comprising a sampling portion (2) in which a liquid sample is collected, a vapor-liquid equilibrium portion (3) in which the liquid substance sample is brought to a vapor-liquid equilibrium at a predetermined temperature and pressure by bubbling purge gas in a vapor-liquid separation vessel (7) to produce a gaseous fraction with said purge gas circulating through a closed flow path, a vapor-liquid separating portion (4) in which the resulting gaseous and liquid fractions are separated from each other, a gaseous fraction expelling portion from which the gaseous fraction is expelled when stopping the circulation after a predetermined time, a gas analytical apparatus (80) into which the gaseous fraction is fed by inflowing carrier gas to analyse it, and a liquid fraction expelling portion which expells the liquid fraction into a liquid analytical apparatus (81) to analyze it.

## Patentansprüche

1. Methode für die Analyse von flüssigen Stoffen, wie zum Beispiel Schmierölen, bei der eine vorbestimmte Menge einer flüssigen Probe abgenommen und die Probe bei einer vorbestimmten Temperatur und bei einem vorbestimmten Druck in Bezug auf Dampf und Flüssigkeit ausgeglichen wird, indem man ein Spülgas in einen Trennkessel für Dampf und Flüssigkeit einbläst, um mit diesem Spülgas, das in einem geschlossenen Fließpfad zirkuliert, eine gasförmige Fraktion herzustellen und die resultierenden gasförmigen und flüssigen Fraktionen voneinander abtrennt, indem man die gasförmige Fraktion dadurch austreibt, daß die Zirkulation nach einer bestimmten Zeit unterbrochen und die gasförmige Fraktion mit Hilfe eines einfließenden Trägergases in ein Gasanalysegerät eingeleitet wird, um die gasförmige Fraktion als Gasprobe und die flüssige Fraktion als Flüssigkeitsprobe zu analysieren.

2. Methode nach Anspruch 1,
**dadurch gekennzeichnet**, **daß** die flüssige Fraktion vor Durchführung der Analyse verdünnt wird.

3. Vorrichtung für die Analyse von flüssigen Substanzen, wie zum Beispiel Schmierölen, die einen Probennahmeteil (2) enthält, in dem eine flüssige Probe angesammelt wird, sowie einen Ausgleichsteil (3) für Dampf und Flüssigkeit, in dem die Probe der flüssigen Substanz bei einer vorbestimmten Temperatur und einem vorbestimmten Druck in ein Gleichgewicht zwischen Dampf und Flüssigkeit gebracht wird, indem ein Spülgas in einen Trennkessel (7) für Dampf und Flüssigkeit eingeblasen wird, um eine gasförmige Fraktion mit dem Spülgas zu bilden, das in einem geschlossenen Pfad zirkuliert, sowie mit einem Trennteil (4) für die Trennung von Dampf und Flüssigkeit, in dem die resultierenden gasförmigen und flüssigen Fraktionen voneinander getrennt werden, und mit einem die gasförmige Fraktion austreibenden Teil, in dem die gasförmige Fraktion ausgetrieben wird, wenn die Zirkulation nach einer vorbestimmten Zeit unterbrochen wird, sowie mit einer Vorrichtung für die Gasanalyse (80), in die die gasförmige Fraktion durch ein einfließendes Trägergas zum Zwecke der Analyse eingeleitet wird, und mit einem die flüssige Fraktion austreibenden Teil, welcher die flüssige Fraktion in eine Flüssigkeitsanalysevorrichtung (81) einleitet, um sie zu analysieren.

## Revendications

1. Procédé d'analyse de substances liquides analogues à une huile de lubrification, comportant les étapes consistant à échantillonner une quantité prédéterminée d'un échantillon de liquide, amener l'échantillon à un état d'équilibre vapeur-liquide à une température et une pression prédéterminées par barbotage d'un gaz de purge dans un réservoir de séparation vapeur-liquide pour produire une traction gazeuse à l'aide dudit gaz de purge circulant à travers un trajet d'écoulement fermé, séparer l'une de l'autre les fractions gazeuses et liquides résultantes par expulsion de la fraction gazeuse en arrêtant la circulation après un temps prédéterminé et en alimentant la fraction gazeuse par un gaz porteur d'introduction dans un dispositif d'analyse de gaz pour analyser la fraction gazeuse en tant qu'échantillon de gaz et la fraction liquide en tant qu'échantillon liquide.

2. Procédé selon la revendication 1, caractérisé en ce que la fraction liquide est diluée avant d'être analysée.

3. Dispositif pour analyser des substances liquides analogues à une huile de lubrification, comportant une partie (2) d'échantillonnage dans laquelle un échantillon de liquide est collecté, une partie (3) de mise en équilibre vapeur-liquide dans laquelle l'échantillon de substance liquide est amené à un état d'équilibre vapeur-liquide à une température et une pression prédéterminées par l'intermédiaire d'un gaz de purge barbotant dans un réservoir (7) de séparation vapeur-liquide pour produire une fraction gazeuse à l'aide dudit gaz de purge circulant à travers un trajet d'écoulement fermé, une partie (4) de séparation vapeur-liquide dans laquelle les fractions gazeuses et liquides résultantes sont séparées l'une de l'autre, une partie d'expulsion de fractions gazeuse à partir de laquelle la fraction gazeuse est expulsée lorsqu'on arrête la circulation après un temps prédéterminé, un dispositif (80) d'analyse de gaz dans lequel la fraction gazeuse est alimentée pour être analysée par introduction d'un gaz porteur, et une partie d'expulsion de fraction liquide qui expulse la fraction liquide jusque dans un dispositif (81) d'analyse liquide pour y être analysée.
